# EUROPEAN PATENT SPECIFICATION

(11) **EP 2 168 943 B1**
(45) Date of publication and mention of the grant of the patent: **12.08.2015**
(21) Application number: 08765705.2
(22) Date of filing: 18.06.2008
(51) Int. Cl.: C07C 213/10, C07C 59/64, C07C 215/44, C07B 57/00, C07C 51/02, C07C 51/41

(54) **METHOD FOR PRODUCING OPTICALLY ACTIVE TRANS-2-AMINOCYCLOHEXANOL AND INTERMEDIATE OF OPTICALLY ACTIVE TRANS-2-AMINOCYCLOHEXANOL**
VERFAHREN ZUR HERSTELLUNG VON OPTISCH AKTIVEM TRANS-2-AMINOCYCLOHEXANOL UND ZWISCHENPRODUKT DES OPTISCH AKTIVEN TRANS-2-AMINOCYCLOHEXANOLS
PROCÉDÉ DE PRODUCTION DU TRANS-2-AMINOCYCLOHEXANOL OPTIQUEMENT ACTIF ET D'UN INTERMÉDIAIRE DU TRANS-2-AMINOCYCLOHEXANOL OPTIQUEMENT ACTIF

(30) Priority: 19.06.2007 JP 2007161963
(43) Date of publication of application: 31.03.2010
(73) Proprietor: Toray Fine Chemicals Co., Ltd., Tokyo 101-0041 (JP)
(72) Inventor: OGAWA, Ryuji, Nagoya-shi Aichi 455-8502 (JP); FUJINO, Toshihiro, Nagoya-shi Aichi 455-8502 (JP); SAKAI, Kenichi, Nagoya-shi Aichi 455-8502 (JP)
(74) Representative: Kador & Partner
(86) International application number: PCT/JP2008/061088
(87) International publication number: WO 2008/156095

(56) References cited:
- JP-A- 09 059 252
- JP-A- 11 060 543
- JP-A- 2001 139 528
- SAKAI K. ET AL.: 'Resolution of 1-cyclohexylethylamine via diastereomeric salt formation' TETRAHEDRON ASYMMETRY vol. 17, no. 10, 19 June 2006, pages 1541 - 1543, XP005526156

## Description

### TECHNICAL FIELD

The invention relates to a method of producing optically active trans-2-aminocyclohexanol from racemic trans-2-aminocyclohexanol.

### BACKGROUND ART

Optically active trans-2-aminocyclohexanol is a compound useful as a raw material for pharmaceuticals and agricultural chemicals.

For example, a known method for producing optically active trans-2-aminocyclohexanol includes subjecting a racemic trans-2-aminocyclohexanol derivative to asymmetric hydrolysis with an enzyme (Patent Literature 1).

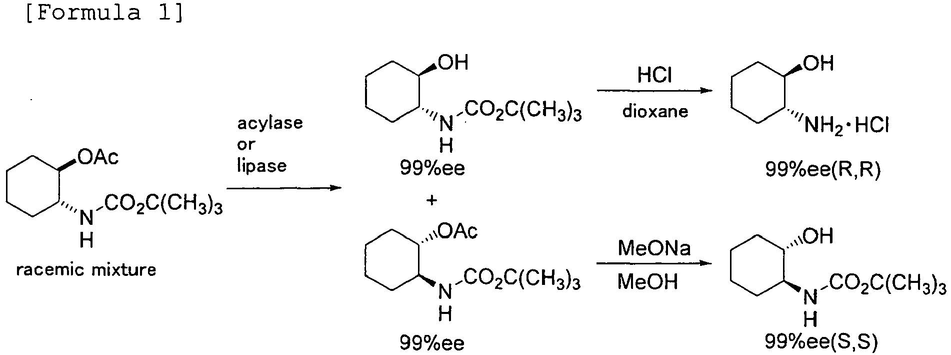

This method includes treating the acetate with an acylase or lipase, separating the respective optically-active products by column chromatography, and then converting the product into optically active trans-2-aminocyclohexanol by hydrolysis with hydrochloric acid. This reaction allows optical resolution at a high optical purity. However, this method is difficult to perform on an industrial production scale, because the asymmetric hydrolysis reaction liquid is a very dilute solution so that the productivity is low. In order to use this method, racemic trans-2-aminocyclohexanol has to be turned into a derivative. Therefore, this method is not considered to be an efficient synthesis method.

A known method for separating diastereomeric salts includes using an optically active organic acid such as optically active di-O-benzoyltartaric acid for optical resolution of racemic trans-2-aminocyclohexanol (Patent Literature 2). In the method specifically disclosed in this literature, however, the optical purity of the optically active trans-2-aminocyclohexanol is about 80% ee, even after crystallization is performed tree times. According to this method, therefore, it is difficult to obtain optically active trans-2-aminocyclohexanol with a satisfactory level of optical purity.

Another known method for separating diastereomeric salts includes using a pine resin-derived optically-active carboxylic acid, dehydroabietic acid, as a resolving agent (Non-Patent Literature 1). However, this method is unsatisfactory in industrial use, because the resolving agent is not industrially or inexpensively available and because neither yield nor selectivity results in a satisfactory level.
Patent Literature 1: Japanese Patent No. 2846770 (Example 3)
Patent Literature 2: Japanese Patent Application Laid-Open (JP-A) No. 09-59252 (Example 1)
Non-Patent Literature 1: Tetrahedron Asymmetry, Vol. 14, pp. 3297-3300, 2003.

### DISCLOSURE OF THE INVENTION

### Problems to be Solved by the Invention

As described above, conventional techniques cannot achieve simple and high yield production of optically active trans-2-aminocyclohexanol under present circumstances, and the development of an efficient industrial process for its production has been strongly demanded.

An object of the invention is to provide a method for producing optically active trans-2-aminocyclohexanol with ease and a high yield from an industrially-advantageous, inexpensive raw material.

### Means for Solving the Problems

As a result of intensive investigations to solve the problems, the inventors have made the invention based on the finding that the reaction of racemic trans-2-aminocyclohexanol with optically active 2-methoxyphenylacetic acid followed by separation of the resulting optically active 2-methoxyphenylacetic acid salt of optically active trans-2-aminocyclohexanol allows efficient production of optically active trans-2-aminocyclohexanol.

Thus, an embodiment of the invention provides a method of producing optically active trans-2-aminocyclohexanol, including: allowing racemic trans-2-aminocyclohexanol represented by the general formula (2): 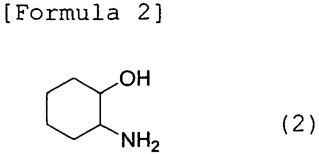 to react with optically active 2-methoxyphenylacetic acid represented by the general formula (3): 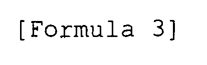 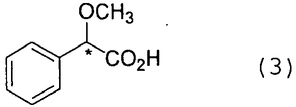 wherein the mark * means that the carbon atom to which the mark is attached is an asymmetric center, to produce an optically active 2-methoxyphenylacetic acid salt of optically active trans-2-aminocyclohexanol; and separating the optically active 2-methoxyphenylacetic acid salt of optically active trans-2-aminocyclohexanol.

Another embodiment of the invention provides a novel compound that is the diastereomeric salt formed in the course of the above optical resolution process.

### Effects of the Invention

According to the invention, optically active trans-2-aminocyclohexanol can be easily produced at a high yield from an industrially-advantageous, inexpensive raw material.

### BEST MODE FOR CARRYING OUT THE INVENTION

The invention is specifically described below. For example, the starting material, trans-2-aminocyclohexanol may be produced according to the reaction formula below, while it may be produced by any appropriate method.

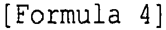

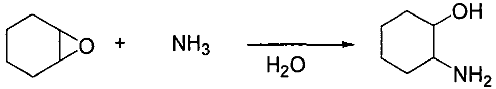

According to the formula, cyclohexene oxide and aqueous ammonia are allowed to react in the presence or absence of a catalyst so that racemic trans-2-aminocyclohexanol can be produced (Tetrahedron Asymmetry, Vol. 14, pp. 3297-3300, 2003). When the reaction is performed with an excess amount of ammonia (10 or more equivalents to one equivalent of cyclohexene oxide) in an autoclave, racemic trans-2-aminocyclohexanol can be produced at a high yield and a high purity. After the reaction, the produced racemic trans-2-aminocyclohexanol may be purified by distillation, crystallization or any other process so that a higher purity product can be obtained. Alternatively, the reaction liquid may be subjected, as it is, to the optical resolution process.

In an embodiment of the invention, optically active 2-methoxyphenylacetic acid for use as an optical resolving agent preferably comprises one enantiomer in an excess of 95% or more, and therefore preferably has an optical purity of 95% ee or more.

For example, the optically active 2-methoxyphenylacetic acid may be produced according to the following reaction formula:

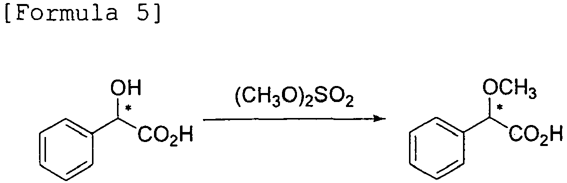

According to the formula, optically active mandelic acid is allowed to react with dimethyl sulfate. After the reaction is completed, the reaction liquid is concentrated, and the precipitated crystal is separated by filtration and then dried to give optically active 2-methoxyphenylacetic acid. The optically active 2-methoxyphenylacetic acid is also commercially available from Yamakawa Chemical Industry Co., Ltd. and industrially available.

The amount of the optically active 2-methoxyphenylacetic acid to be used is preferably 0.5 to 2.0 times, more preferably 0.9 to 1.1 times, by mole, the amount of racemic trans-2-aminocyclohexanol. In addition, the optically active 2-methoxyphenylacetic acid may be used in combination with an inorganic acid such as hydrochloric acid or sulfuric acid or an optically inactive material such as acetic acid or propionic acid. In such a case, the amount of the optically active 2-methoxyphenylacetic acid to be used can be reduced.

A solvent non-reactive with the substrate should be used in the optical resolution. Examples of such a solvent that is preferably used include water, alcohols such as methanol and ethanol, nitriles such as acetonitrile, and ethers such as tetrahydrofuran. One or more of these solvents may be used alone or in the form of a mixed solvent. In particular, water, methanol, ethanol, propanol, or any mixture thereof is preferred. In view of workability and safety, water is preferably used.

The amount of the solvent to be used is preferably 1.0 to 20.0 times, in particular, preferably 2.0 to 10.0 times, by weight, the amount of racemic trans-2-aminocyclohexanol.

The temperature of the optical resolution is generally in the range of 0°C to below the boiling point of the solvent, preferably 20 to 80°C, while it depends on the type of the solvent.

The optical resolution may be performed by a method including mixing the starting material racemic trans-2-aminocyclohexanol, the optically active 2-methoxyphenylacetic acid and the solvent and separating the precipitated salt by filtration. In this case, examples of the mixing method include, but are not limited to, a method of mixing the materials at once, a method including mixing the starting material racemic trans-2-aminocyclohexanol and the solvent and then adding the optically active 2-methoxyphenylacetic acid thereto under stirring, and a method including alternatively mixing the solvent and the optically active 2-methoxyphenylacetic acid and then adding the starting material racemic trans-2-aminocyclohexanol thereto under stirring. In view of workability, the method of mixing the materials at once is preferred. After the mixing process, the mixture may be heated to form a solution or brought into sufficient equilibrium in a slurry state. The heating temperature is preferably, but not limited to, 30°C to 100°C, particularly preferably 40°C to 80°C, in view of workability. After heating and aging, the temperature may be gradually lowered, and the precipitated crystal may be filtered off and isolated. The lowered temperature for crystallization is preferably, but not limited to, -10°C to 40°C, particularly preferably 10°C to 30°C, in view of workability. When the adhesion of the mother liquor has a significant effect or when a particularly high optical purity product is produced, the solvent may be added again to the crystal to form a solution or a slurry for washing, and after cooling, a crystal of the precipitated optically active 2-methoxyphenylacetic acid salt of optically active trans-2-aminocyclohexanol may be filtered off, so that a high optical purity can be easily achieved. In this process, water, methanol, ethanol, or the like is preferably used as the solvent.

According to conventional methods, the resulting optically active 2-methoxyphenylacetic acid salt of optically active trans-2-aminocyclohexanol may be decomposed so that optically active 2-aminocyclohexanol can be isolated. For example, optically active trans-2-aminocyclohexanol may be obtained by a process including: adding the optically active 2-methoxyphenylacetic acid salt of optically active trans-2-aminocyclohexanol to a mixed solution of water and hydrochloric acid; separating the precipitated crystal by filtration to remove optically active 2-methoxyphenylacetic acid; alkalinizing the filtrate; and then extracting optically active trans-2-aminocyclohexanol with an organic solvent. The organic solvent to be used for the extraction is preferably an alcohol such as methanol, ethanol, propanol, or 1-butanol, a ketone such as acetone or methyl ethyl ketone, an ester such as ethyl acetate or butyl acetate, an ether such as diethyl ether, tetrahydrofuran or digrime, a hydrocarbon such as hexane, toluene or xylene, a halogen-containing solvent such as dichloromethane or chloroform, or the like. In particular, chloroform, 1-butanol or the like is preferably used in view of solubility. One or more of these solvents may be used alone or in the form of a mixed solvent.

An alternative process may include: adding the optically active 2-methoxyphenylacetic acid salt of optically active trans-2-aminocyclohexanol to a mixed solution of water and a protic acid; separating the precipitated crystal by filtration to remove optically active 2-methoxyphenylacetic acid; concentrating the filtrate; and separating the precipitated crystal by filtration so that a protic acid salt of optically active trans-2-aminocyclohexanol can be isolated. Examples of the protic acid include mineral acids such as hydrochloric acid, sulfuric acid and phosphoric acid; carboxylic acids such as formic acid, acetic acid, propionic acid, and citric acid; and sulfonic acids such as methanesulfonic acid and p-toluenesulfonic acid.

The method of the invention makes it possible to produce optically active 2-aminocyclohexanol at a high yield and a high purity using inexpensively producible racemic trans-2-aminocyclohexanol as a starting material and using industrially available optically active 2-methoxyphenylacetic acid.

The optically active trans-2-aimnocyclohexanol obtained as described above is a useful compound as a raw material for pharmaceuticals and agricultural chemicals.

### EXAMPLES

The invention is more specifically described by the examples below, which are not intended to limit the scope of the invention.

In the examples, R- and S-2-methoxyphenylacetic acid (with an optical purity of at least 99% ee) used was manufactured by Yamakawa Chemical Industry Co., Ltd.

In the examples, the chemical purity of trans-2-aminocyclohexanol was determined by GC analysis.

### <Analysis (GC) of Chemical Purity of Trans-2-Aminocyclohexanol>

Column: TC-17 (GL Science Inc.) 60 m-0.32 mm I.D. 0.25 µm Temperature: 70°C (10 minutes) → 20°C/minute → 270°C (10 minutes)
Inlet: 200°C
Detector: 200°C

The optical purity of trans-2-aminocyclohexanol was determined by HPLC analysis, after it was labeled with 2,3,4,6-tetra-O-acetyl-β-D-glucopyranosyl isothiocyanate (GITC, manufactured by Wako Pure Chemical Industries, Ltd.).

### <Optical Purity Analysis (HPLC)>

Column: CAPCELL PAK C18 SG-120 (Shiseido Co., Ltd.) 150 mm-4.6 mmφ (5 µm)
Mobile phase: liquid A, an aqueous 5 mM sodium lauryl sulfate solution (pH 2.20); liquid B, acetonitrile; A/B = 80/20
Flow rate: 1.0 ml/minute
Detector: UV 243 nm
Temperature: 40°C

### Reference Example 1 (Synthesis of Racemic Trans-2-Aminocyclohexanol)

To a 1 L-volume autoclave equipped with a stirrer were added 98.1 g (1 mol) of cyclohexene oxide and 608.2 g (10 mol) of an aqueous 28% ammonia solution, and stirred at 60 to 65°C for 4 hours. After the mixture was cooled to room temperature, the precipitated crystal (2-(2-hydroxycyclohexyl)aminocyclohexanol) was removed by filtration, and the ammonia was concentrated under normal pressure. The reaction liquid was then concentrated to about 100 g under reduced pressure. After 290 g of toluene was added to the concentrate, the mixture was concentrated again. The precipitated crystal was separated by filtration and dried under reduced pressure to give 75.3 g of racemic trans-2-aminocyclohexanol (64.0% yield).

### Example 1 (Optical Resolution of Racemic Trans-2-Aminocyclohexanol with R-Methoxyphenylacetic Acid)

To a 20 ml-volume sample vial with a stopper were added 0.17 g (1.5 mmol) of racemic trans-2-aminocyclohexanol obtained in Reference Example 1, 0.25 g (1.5 mmol) of R-2-methoxyphenylacetic acid and 2.0 ml of water, and then heated to 60°C to form a solution. The solution was cooled to 20 to 23°C, and the precipitated crystal was separated by filtration and dried to give 0.09 g of a salt. The resulting (1S,2S)-trans-2-aminocyclohexanol had an optical purity of 97% ee.

### Example 2 (Optical Resolution of Racemic Trans-2-Aminocyclohexanol with R-2-Methoxyphenylacetic Acid)

To a 20 ml-volume sample vial with a stopper were added 0.17 g (1.5 mmol) of racemic trans-2-aminocyclohexanol obtained in Reference Example 1, 0.25 g (1.5 mmol) of R-2-methoxyphenylacetic acid and 2.0 ml of methanol, and then heated to 60°C to form a solution. The solution was cooled to 20 to 23°C, and the precipitated crystal was separated by filtration and dried to give 0.15 g of a salt. The resulting (1S,2S)-trans-2-aminocyclohexanol had an optical purity of 91% ee.

### Example 3 (Optical Resolution of Racemic Trans-2-Aminocyclohexanol with R-2-Methoxyphenylacetic Acid)

To a 100 ml-volume four-neck flask equipped with a stirrer, a thermometer and a condenser were added 5.76 g (50 mmol) of racemic trans-2-aminocyclohexanol obtained in Reference Example 1, 8.31 g (50 mmol) of R-2-methoxyphenylacetic acid and 32.4 g of water, and heated to 80°C. The mixture was aged at 70°C for 1 hour and then cooled to 20 to 25°C over 3 hours. After the mixture was stirred at the same temperature for 1 hour, the precipitated crystal was separated by filtration. The crystal was rinsed with 5.0 g of water and then dried to give 4.72 g of a salt. The content of trans-2-aminocyclohexanol in the salt was 40.9%. The resulting (1S,2S)-trans-2-aminocyclohexanol had an optical purity of 99.6% ee and a yield of 67.1%.

### Example 4 (Optical Resolution of Racemic Trans-2-Aminocyclohexanol with R-2-Methoxyphenylacetic Acid)

To a 100 ml-volume four-neck flask equipped with a stirrer, a thermometer and a condenser were added 5.76 g (50 mmol) of racemic trans-2-aminocyclohexanol obtained in Reference Example 1, 8.31 g (50 mmol) of R-2-methoxyphenylacetic acid and 26.1 g of water, and heated to 80°C. The mixture was aged at 70°C for 1 hour and then cooled to 20 to 25°C over 3 hours. After the mixture was stirred at the same temperature for 1 hour, the precipitated crystal was separated by filtration. The crystal was rinsed with 3.9 g of water and then dried to give 5.53 g of a salt. The content of trans-2-aminocyclohexanol in the salt was 40.9%. The resulting (1S,2S)-trans-2-aminocyclohexanol had an optical purity of 97.2% ee and a yield of 77.5%.

### Example 5 (Optical Resolution of Racemic Trans-2-Aminocyclohexanol with R-2-Methoxyphenylacetic Acid)

To a 100 ml-volume four-neck flask equipped with a stirrer, a thermometer and a condenser were added 2.88 g (25 mmol) of racemic trans-2-aminocyclohexanol obtained in Reference Example 1, 4.15 g (25 mmol) of R-2-methoxyphenylacetic acid and 28.1 g of water, and heated to 60°C. The mixture was aged at 55°C for 1 hour and then cooled to 20 to 25°C over 3 hours. After the mixture was stirred at the same temperature for 1 hour, the precipitated crystal was separated by filtration. The crystal was rinsed with 5.1 g of water and then dried to give 1.50 g of an R-2-methoxyphenylacetic acid salt of (1S,2S)-trans-2-aminocyclohexanol. The content of trans-2-aminocyclohexanol in the salt was 40.9%. The resulting (1S,2S)-trans-2-aminocyclohexanol had an optical purity of 99.8% ee and a yield of 42.7%.
¹H-NMR (400 MHz, D₂O)δ: 7.22-7.29 (m, 5H), 4.48 (s, 1H), 3.32 (dt, 1H, J = 4.0 Hz, 10.2 Hz), 3.20 (s, 3H), 2.77 (dt, 1H, J = 4.4 Hz, 10.8 Hz), 1.86 (d, 2H, J = 12.0 Hz), 1.56 (d, 2H, J = 5.6 Hz), 1.06-1.25 (m, 4H).
Specific rotation [α]_{D} = -4.9° (c = 5, water, 25°C).
Melting point 189-191°C (decomposition).

The filtration mother liquor was concentrated, and the precipitated crystal was separated by filtration. The resulting crystal was repeatedly recrystallized to give an R-2-methoxyphenylacetic acid salt of (1R,2R)-trans-2-aminocyclohexanol with an optical purity of at least 99.5% ee.
¹H-NMR (400 MHz, D₂O)δ: 7.24-7.31 (m, 5H), 4.50 (s, 1H), 3.34 (dt, 1H, J = 4.0 Hz, 10.4 Hz), 3.22 (s, 3H), 2.79 (dt, 1H, J = 4.0 Hz, 11.0 Hz), 1.86 (d, 2H, J = 11.6 Hz), 1.58 (d, 2H, J = 6.4 Hz), 1.08-1.24 (m, 4H).
Specific rotation [α]_{D} = -9.3° (c = 5, water, 25°C).
Melting point 159-161°C (decomposition).

### Example 6 (Optical Resolution of Racemic Trans-2-Aminocyclohexanol with S-2-Methoxyphenylacetic Acid)

To a 200 ml-volume four-neck flask equipped with a stirrer, a thermometer and a condenser were added 11.52 g (100 mmol) of racemic trans-2-aminocyclohexanol obtained in Reference Example 1, 16.62 g (25 mmol) of S-2-methoxyphenylacetic acid and 42.2 g of methanol, and heated to 70°C. The mixture was aged at 65°C for 1 hour and then cooled to 20 to 25°C over 3 hours. After the mixture was stirred at the same temperature for 1 hour, the precipitated crystal was separated by filtration. The crystal was rinsed with 10.0 g of methanol and then dried to give 12.58 g of a salt. The content of trans-2-aminocyclohexanol in the salt was 40.9%. The resulting (1R,2R)-trans-2-aminocyclohexanol had an optical purity of 76.2% ee and a yield of 78.8%. To a 100 ml-volume four-neck flask equipped with a stirrer, a thermometer and a condenser were added 12.27 g (44 mmol) of the resulting crystal and 37.5 g of methanol, and heated to 70°C. The mixture was aged at 65°C for 1 hour and then cooled to 20 to 25°C over 3 hours. After the mixture was stirred at the same temperature for 1 hour, the precipitated crystal was separated by filtration. The crystal was rinsed with 4.4 g of methanol and then dried to give 9.67 g of an S-2-methoxyphenylacetic acid salt of (1R,2R)-trans-2-aminocyclohexanol. The optical purity of the (1R,2R) isomer was at least 99% ee.
¹H-NMR (400 MHz, D₂O)δ: 7.22-7.29 (m, 5H), 4.48 (s, 1H), 3.32 (dt, 1H, J = 4.0 Hz, 10.2 Hz), 3.20 (s, 3H), 2.77 (dt, 1H, J = 4.4 Hz, 10.8 Hz), 1.86 (d, 2H, J = 12.0 Hz), 1.56 (d, 2H, J = 5.6 Hz), 1.06-1.25 (m, 4H).
Specific rotation [α]_{D} = 4.7° (c = 5, water, 25°C).
Melting point 189-191°C (decomposition).

The filtration mother liquor was concentrated, and the precipitated crystal was separated by filtration. The resulting crystal was repeatedly recrystallized to give an S-2-methoxyphenylacetic acid salt of (1S,2S)-trans-2-aminocyclohexanol with an optical purity of at least 99.5% ee.
¹H-NMR (400 MHz, D₂O)δ: 7.24-7.31 (m, 5H), 4.50 (s, 1H), 3.34 (dt, 1H, J = 4.0 Hz, 10.4 Hz), 3.22 (s, 3H), 2.79 (dt, 1H, J = 4.0 Hz, 11.0 Hz), 1.86 (d, 2H, J = 11.6 Hz), 1.58 (d, 2H, J = 6.4 Hz), 1.08-1.24 (m, 4H).
Specific rotation [α]_{D} = 8.3° (c = 5, water, 25°C).
Melting point 159-161°C (decomposition).

### Example 7 (Decomposition of R-Methoxyphenylacetic Acid Salt of (1S,2S)-Trans-2-Aminocyclohexanol)

To a 2,000 ml-volume four-neck flask equipped with a stirrer, a thermometer and a condenser were added 31.4 g of an R-2-methoxyphenylacetic acid salt of (1S,2S)-trans-2-aminocyclohexanol obtained by the same method as in Example 3 ((1S,2S)-trans-2-aminocyclohexanol: content, 40.9%; 11.6 g; 101 mmol), 62.7 g of water and 62.7 g of toluene. Under stirring, 12.7 g (122 mmol) of an aqueous 35% hydrochloric acid solution was added to the mixture to adjust the pH to 2.0. After fractionation, the toluene layer was removed, and the water layer was washed again with 62.7 g of toluene. The resulting water layer was mixed with 181.1 g of 1-butanol. Under stirring, 11.1 g (134 mmol) of an aqueous 48% sodium hydroxide solution was added to the mixture to adjust the pH to 10.5. After fractionation, the organic layer was taken out, and the water layer was extracted again with 80.4 g of 1-butanol. The resulting organic layers were combined and concentrated using an evaporator. To the concentrate was added 117.6 g of toluene, and the precipitated inorganic salt was separated by filtration. While toluene was added, the filtrate was further concentrated using a vacuum concentrator, so that a crystal was precipitated. After cooling to room temperature, the precipitated crystal was separated by filtration and then dried, so that 10.2 g of (1S,2S)-trans-2-aminocyclohexanol was obtained as a white solid. The (1S,2S)-trans-2-aminocyclohexanol had a chemical purity of 99.8%, an optical purity of at least 99.5% ee, and an optical rotation [α]_{D} of -40.2° (c = 0.4, acetonitrile, 23°C).
¹H-NMR (400 MHz, CDCl₃)δ: 3.10-3.15 (m, 1H), 2.40-2.46 (m, 1H), 2.23 (br, 3H), 1.67-1.97 (m, 4H), 1.23-1.27 (m, 3H), 1.10-1.16 (m, 1H)
¹³C-NMR (400 MHz, CDCl₃) δ: 75.8, 57.0, 34.8, 33.7, 25.1, 24.8

### Comparative Example 1 (Optical Resolution of Racemic Trans-2-Aminocyclohexanol with Dibenzoyl-L-Tartaric Acid)

To a 1,000 ml-volume four-neck flask equipped with a stirrer, a thermometer and a condenser were added 25.4 g (221 mmol) of racemic trans-2-aminocyclohexanol obtained by the same method as in Reference Example 1, 41.4 g (110 mmol) of dibenzoyl-L-tartaric acid monohydrate, 60 g of methanol, and 360 g of ethanol, and heated to 90°C. After the mixture was cooled to 70°C and aged for 1 hour, it was cooled to 20 to 25°C over 5 hours and then stirred at the same temperature for 1 hour. The precipitated crystal was separated by filtration and dried under reduced pressure to give 36.7 g of a primary crystal. This process was performed once again, so that 23.1 g of a secondary crystal was obtained. The content of trans-2-benzylaminocyclohexanol in the salt was 24.3%. The resulting (1R,2R)-trans-2-benzylaminocyclohexanol had an optical purity of 69.2% ee and a yield of 37.5%.

## Claims

1. A method of producing optically active trans-2-aminocyclohexanol, comprising:
allowing racemic trans-2-aminocyclohexanol to react with optically active 2-methoxyphenylacetic acid to produce an optically active 2-methoxyphenylacetic acid salt of optically active trans-2-aminocyclohexanol; and
separating the optically active 2-methoxyphenylacetic acid salt of optically active trans-2-aminocyclohexanol.

2. The method according to Claim 1, wherein water is used as a solvent in the separating.

3. An optically active 2-methoxyphenylacetic acid salt of optically active trans-2-aminocyclohexanol represented by the general formula (1): 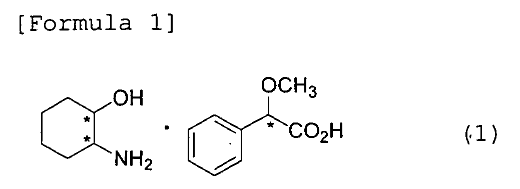 wherein the mark * means that the carbon atom to which the mark is attached is an asymmetric center.

## Patentansprüche

1. Verfahren zur Herstellung von optisch aktivem trans-2-Aminocyclohexanol, umfassend:
Ermöglichen von razemischem trans-2-Aminocyclohexanol mit optisch aktiver 2-Methoxyphenylessigsäure zu reagieren, um ein optisch aktives 2-Methoxyphenylessigsäuresalz des optisch aktiven trans-2-Aminocyclohexanols herzustellen; und
Trennen des optisch aktiven 2-Methoxyphenylessigsäuresalzes von optisch aktivem trans-2-Aminocyclohexanol.

2. Verfahren nach Anspruch 1, wobei bei der Trennung Wasser als das Lösungsmittel verwendet wird.

3. Optisch aktives 2-Methoxyphenylessigsäuresalz des optisch aktiven trans-2-Aminocyclohexanols, repräsentiert durch die allgemeine Formel (1): 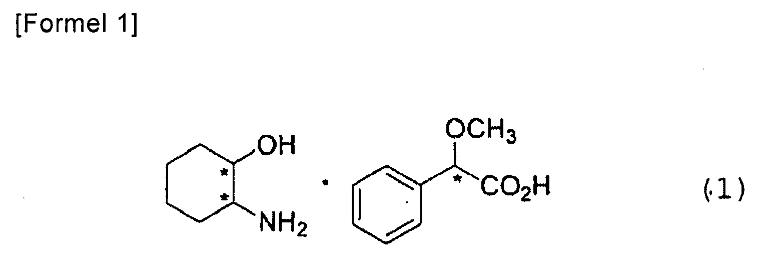 wobei die Markierung * bedeutet, dass das Kohlenstoffatom, an dem sich die Markierung befindet, ein asymmetrisches Zentrum ist.

## Revendications

1. Procédé de production de trans-2-aminocyclohexanol optiquement actif, comprenant :
la réaction de trans-2-aminocyclohexanol racémique avec de l'acide 2-méthoxyphénylacétique optiquement actif pour produire un sel d'acide 2-méthoxyphénylacétique optiquement actif de trans-2-aminocyclohexanol optiquement actif ; et
la séparation du sel d'acide 2-méthoxyphénylacétique optiquement actif de trans-2-aminocyclohexanol optiquement actif.

2. Procédé selon la revendication 1, dans lequel de l'eau est utilisée en tant que solvant dans la séparation.

3. Sel d'acide 2-méthoxyphénylacétique optiquement actif de trans-2-aminocyclohexanol optiquement actif représenté par la formule générale (1) : 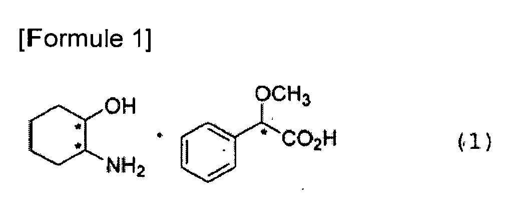 dans lequel la marque * signifie que l'atome de carbone auquel la marque est associée est un centre asymétrique.
